# EUROPEAN PATENT SPECIFICATION

(11) **EP 3 970 973 B1**
(45) Date of publication and mention of the grant of the patent: **20.12.2023**
(21) Application number: 21197053.8
(22) Date of filing: 16.09.2021
(51) Int. Cl.: B41F 5/24, B41F 19/00, B65D 27/00, D21H 21/36, B65B 55/10

(54) **METHOD OF MANUFACTURING A SANITISED PAPER ENVELOPE**
VERFAHREN ZUR HERSTELLUNG EINER DESINFIZIERTEN PAPIERHÜLLE
PROCÉDÉ DE FABRICATION D'UNE ENVELOPPE EN PAPIER ASEPTISÉ

(30) Priority: 18.09.2020 GB 202014763
(43) Date of publication of application: 23.03.2022
(73) Proprietor: Trimfold Envelopes Limited, C15 TY23 Co. Meath (IE)
(72) Inventor: HEALY, Eugene, Trim C15 TY23 (IE)
(74) Representative: Definition IP Limited

(56) References cited:
- WO-A1-2019/117822
- DE-A1-102007 031 580
- FR-A1- 2 722 136
- US-A1- 2004 161 450

## Description

### Technical Field

The present invention relates to a method of manufacturing a sanitised paper envelope using a flexographic printing apparatus.

### Background

Cutlery envelopes are used in the catering industry in order to pre-package individual sets of cutlery for customers. For example, each cutlery envelope contains a knife, fork and spoon as well as any suitable condiments, such as sachets of salt/pepper, and a napkin. Individual envelopes can be supplied to customers prior to dining. Each envelope can therefore contain all of the necessary cutlery and accessories that a customer may require when dining.

Use of a cutlery envelope also reduces the number of times cutlery is handled directly, for example by serving staff, and so offers the added benefit of increasing hygiene. Cutlery envelopes also offer protection from airborne transmission of pathogens from people in the vicinity of where cutlery is handled, stored or laid on a table, for example.

Nevertheless, it is to be appreciated that such cutlery envelopes do not eliminate the risk of contamination, particularly with regard to pathogens that may be prevalent in the catering establishment or which may have come into contact with the cutlery envelopes themselves during manufacture or prior to use.

Therefore, there exists a need to reduce the risk of transmission of pathogens to cutlery used in a catering environment.

FR2722136A relates to a method of manufacture of envelopes by shaping, cutting and folding a strip of paper from a reel. DE 10 2007 031580A relates to an insertion pocket for receiving cutlery, which is made of a paper sheet folded multiple times.

### Summary of the Invention

According to a first aspect of the invention there is provided a method according to claim 1, namely a method of manufacturing a sanitised paper envelope comprising the steps: supplying a paper sheet having a first surface and a second surface to a flexographic printing apparatus having a first sanitising module; applying a sanitising solution to at least the first surface of the paper sheet using the first sanitising module, and forming an envelope from the sanitised paper sheet.

The aim of the present invention is to produce an envelope which reduces the risk of transmission of pathogens, such as a hygienic cutlery envelope.

It will be appreciated that in certain embodiments, a paper envelope could be manufactured from multiple sheets of sanitised paper.

The flexographic printing apparatus may further comprise a second sanitising module and the method further comprises the step of applying a sanitising solution to the second surface of the paper sheet using the second sanitising module.

The step of applying a sanitising solution to the second surface may be subsequent to the step of applying a sanitising solution to the first surface.

The flexographic printing apparatus may further comprise at least one ink printing module and the method may further comprise the step of printing a graphic and/or text on at least one of the first and second surfaces of the paper sheet. The graphic and/or text may, for example, be printed on the first surface of the paper sheet.

The step of printing on at least one of the first and second surfaces of the paper sheet may be subsequent to the step of applying a sanitising solution to at least the first surface of the paper sheet.

The sanitising solution may comprise a sanitising agent and a carrier agent. The carrier agent may be a liquid. The sanitising agent may comprise ethanol. The sanitising solution may comprise at least 70% ethanol. The paper sheet is supplied from a continuous roll of paper. The step of forming the envelope may comprise the steps of cutting a blank having a predetermined shape from the sanitised paper sheet, and configuring said blank to form a pocket having an aperture along one side.

The blank may comprise a back panel, a front panel and a closure panel arranged such that the back panel and the front panel can be folded towards each other to form the pocket and the closure panel can be folded over the aperture to inhibit access to the pocket.

The method may further comprise a step of applying an adhesive covered by a removable protective strip to the blank for securing the closure panel over the aperture.

The method may further comprise a step of forming a perforation extending across a region of the envelope such that a user can remove a portion of the envelope from the remaining envelope by tearing along the perforation.

The sanitising module may comprise a fluid supply unit, an application roller for applying a sanitising solution to a paper sheet and a transfer roller arranged to transfer the sanitising fluid from the fluid supply unit to the application roller.

The sanitising module may further comprise at least one doctor blade arranged to control the amount of sanitising solution transferred by the transfer roller to the application roller.

According to a second aspect of the invention there is provided a sanitised paper envelope according to claim 17, namely a sanitised paper envelope that has been manufactured in accordance with the method Z mentioned above.

### Brief Description of the Drawings

Embodiments of the present invention will now be described by way of example with reference to the accompanying drawings where like parts are provided with corresponding reference numerals and in which:
Figure 1 is a schematic representation of a flexographic printing apparatus;
Figure 2 is a flow chart illustrating process steps of a method manufacturing a sanitised paper article;
Figure 3 is a blank used to form an envelope;
Figure 4A shows a front side of an envelope;
Figure 4B shows a rear side of an envelope;
Figure 5A illustrates the correlation between cell densities and distribution;
Figure 5B shows a comparison between dot sizes; and
Figure 6 is table illustrating technical specifications for transfer rollers.

### Detailed Description

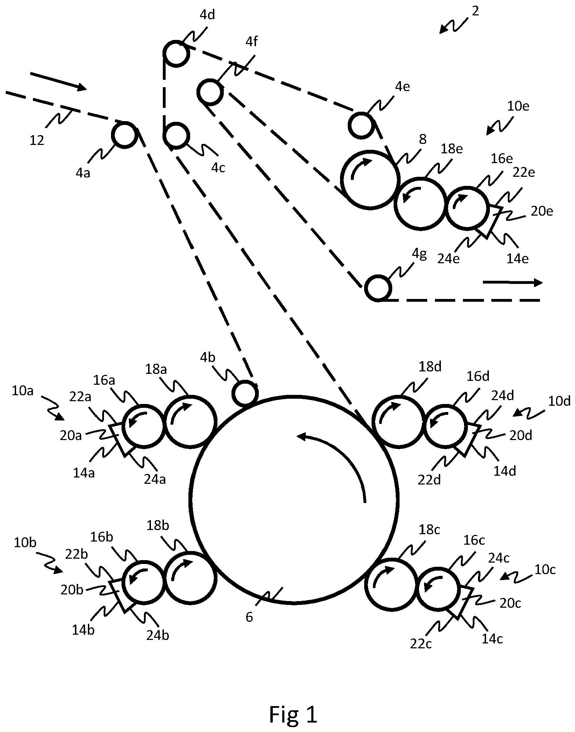

Figure 1 shows a flexographic printing apparatus 2 comprising a plurality of guide rollers 4a, 4b, 4c, 4d, 4e, 4f, 4g, a first impression cylinder 6, a second impression cylinder 8, a first sanitising module 10a, first, second and third printing modules 10b, 10c, 10d, and a second sanitising module 10e.

First and second guide rollers 4a, 4b are arranged to guide a paper sheet 12 (which may also be termed a paper substrate) drawn from a roll (not shown) to an outer surface of the first impression cylinder 6 such that the paper sheet 12 is brought into contact with the first impression cylinder 6.

Second, third and fourth guide rollers 4c, 4d, 4e are arranged to guide the paper sheet 12 from the outer surface of the first impression cylinder 6 to the second impression cylinder 8 such that the paper sheet 12 is brought into contact with an outer surface of the second impression cylinder 8. Fifth and sixth guide rollers 4f, 4g are arranged to guide the paper sheet 12 away from the second impression cylinder 8 for further processing, such as cutting.

The printing modules 10b, 10c, 10d and sanitising modules 10a, 10e have the same construction. Each comprises a respective fluid (e.g. ink or sanitising solution) supply unit 14a, 14b, 14c, 14d, 14e, a respective transfer roller 16a, 16b, 16c, 16d, 16e and a respective application roller 18a, 18b, 18c, 18d, 18e.

Each fluid supply unit 14a, 14b, 14c, 14d, 14e comprises a fluid, and specifically a liquid, reservoir 20a, 20b, 20c, 20d, 20e formed, in part, by spaced apart first and second 'doctor' blades 22a, 22b, 22c, 22d, 22e and 24a, 24b, 24c, 24d, 24e. The doctor blades 22a, 22b, 22c, 22d, 22e, 24a, 24b, 24c, 24d, 24e are arranged to press against an outer surface of a respective transfer roller 16a, 16b, 16c, 16d, 16e so as to seal the reservoir 20a, 20b, 20c, 20d, 20e against the transfer roller 16a, 16b, 16c, 16d, 16e.

Each transfer roller 16a, 16b, 16c, 16d, 16e is a conventional transfer roller (which may also be known as a screen roller, coating roller or anilox roller) configured to collect a layer of liquid from a respective liquid supply unit 14a, 14b, 14c, 14d, 14e having a precise thickness. Each transfer roller 16a, 16b, 16c, 16d, 16e is a ceramic coated roller having a plurality of identical cells on its outer surface which have a predefined volume. The cells may be arranged in any suitable matrix, such as a honeycomb pattern. In the present embodiment each transfer roller 16a, 16b, 16c, 16d, 16e has a cell density of 160 cells/cm², but transfer rollers having cell densities between 100 cells/cm2 and 315 cells/cm² could be used (for applications requiring higher volumes of liquid transfer, lower cell densities are typically used, whereas for applications requiring lower volumes of liquid transfer, higher cell densities are typically used). Each cell is approximately 10cm deep and has a predetermined cell volume in the range 3.5 cm³ to 6 cm³, such as 5 cm³.

In general, the technical specification of a transfer roller determines the print quality. Typically, the objective is to use always the highest cell density which satisfy required colour densities or, where applicable, the required distribution of sanitising solution.

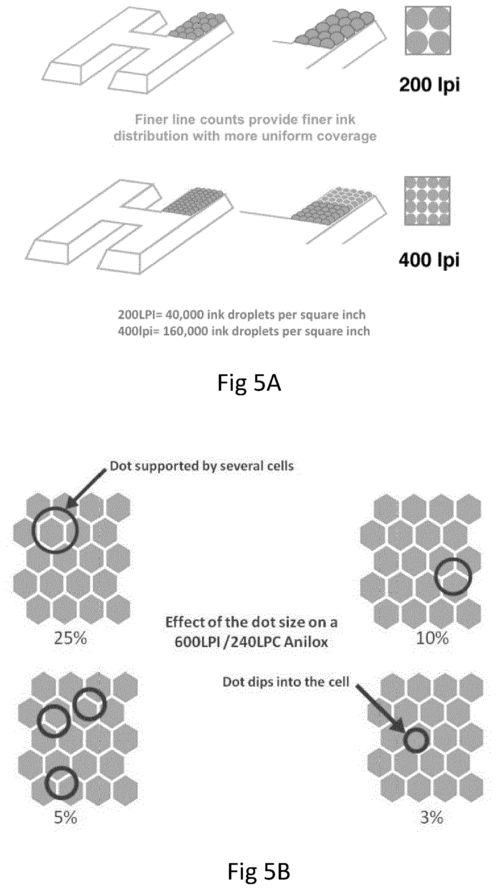

The higher the cell density (which correlates with a line count, as is known in the art of flexographic printing) the finer the ink/sanitising solution distribution, as illustrated by Figure 5A. The cell density determines the total number of ink dots that will be transferred to an application roller. Higher cell densities place more cells, closer together. Smaller dots will dry faster producing less dot gain which will offer better image clarity or a more controlled application of sanitising solution.

When selecting a line count (i.e. cell density) for a process job, it is typical to specify a line count which is five times the desired printing resolution. This ratio allows the cell walls to adequately support the dot and keeps the dot from dipping into the cells, as illustrated by Figure 5B.

Cell density is chosen in correlation with cell volume. For example, if printing solids (i.e. an unbroken image or continuous coating), such as the application of a sanitising solution, a volume of 8cm³/m² is typically required. The cell density is therefore selected accordingly and so the line count could be approximately 400LPI (which equates to 1600,000 cells per square inch).

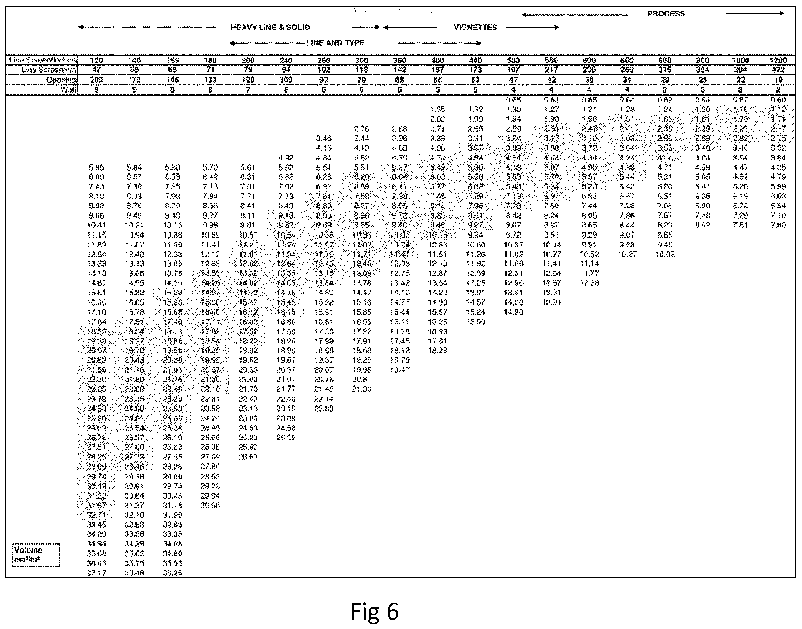

Figure 6 provides a table illustrating example technical specifications for transfer rollers.

Each transfer roller 16a, 16b, 16c, 16d, 16e is arranged to contact a respective application roller 18a, 18b, 18c, 18d, 18e so as to deposit fluid on the application roller 18a, 18b, 18c, 18d, 18e.

Each application roller 18a, 18b, 18c, 18d, 18e is a conventional application roller (also termed a print roller) comprising a cylinder and a flexible plate (e.g. a printing plate or a coating plate) wrapped around the cylinder. In the embodiment shown, the flexible plates are photopolymer print plates. The plates have a shore hardness of approximately 40. Each flexible plate for the application rollers 18b, 18c, 18d of the printing modules 10b, 10c, 10d has portions which define an image (such as a logo) and/or text that is to be printed, as required. The text/images are created on the plates using a conventional process, such as by exposing portions of the plates defining the text/image to an ultraviolet light and then washing uncured regions of the surface away.

The first, second, third and fourth application rollers 18a, 18b, 18c, 18d are arranged circumferentially about the first impression cylinder 6 and spaced apart approximately equidistantly. Each application roller 18a, 18b, 18c, 18d is arranged for pressing engagement with the first impression cylinder 6 such that the flexible plate contacts the paper sheet 12 when the paper sheet 12 is wound around the first impression cylinder 6.

The fifth application roller 18e is arranged for pressing engagement with the second impression cylinder 8 such that the flexible plate contacts the paper sheet 12 when wound around the second impression cylinder 8.

The flexible plates of each of the first and fifth application rollers 18a, 18e are configured to apply a continuous coating of sanitising solution to the paper sheet 12.

Prior to operation, a paper sheet 12 is arranged to extend from a roll of paper (not shown) through the flexographic printing apparatus 2. The paper sheet 12 may be passed through an alignment station prior to reaching the printing apparatus 2 in order to align the paper for printing. The paper sheet 12 extends over the first guide roller 4a and down to the second guide roller 4b and the first impression cylinder 6, where it is brought into contact with an outer pressing surface of the first impression cylinder 6 as it passes between the second guide roller 4b and the first impression cylinder 6.

The paper sheet 12 extends circumferentially around the outer surface of the first impression cylinder 6 and passes between each of the application rollers 18a, 18b, 18c, 18d of the first sanitising module 10a and the first, second and third printing modules 10b, 10c, 10d, and the first impression cylinder 6.

The paper sheet 12 extends away from the outer surface of the first impression cylinder 6 and upwardly towards the third guide roller 4c where it passes around the third guide roller 4c before passing over the fourth guide roller 4d and down over the fifth guide roller 4e to the second impression cylinder 8.

The paper sheet 12 extends circumferentially around the outer surface of the second impression cylinder 8 and passes between the application roller 18e of the second sanitising module 10e and the second impression cylinder 8. The paper sheet 12 then extends upwardly and around the fifth guide roller 4f and then extend back towards and under the sixth roller 4f and away from the printing apparatus 2.

Each reservoir 20a, 20b, 20c, 20d, 20e is either filled with a liquid which is to be applied or else connected to such a liquid supply.

For example, the fluid reservoir 20a of the first sanitising module 10a is filled with a sanitising solution, such as a solution comprising at least 70% ethanol. The sanitising solution is clear in the sense that it has no discernible colour.

The fluid reservoir 20b of the first printing module 10b is filled with a first ink, such as an ink having a desired first colour.

The fluid reservoir 20c of the second printing module 10c is filled with a second ink, such as an ink having a desired second colour.

The fluid reservoir 20d of the third printing module 10d is filled with a third ink, such as an ink having a desired third colour.

The fluid reservoir 20e of the second sanitising module 10e is filled with a sanitising solution, such as a sanitising solution which is the same as the sanitising solution used in the first sanitising module 10a.

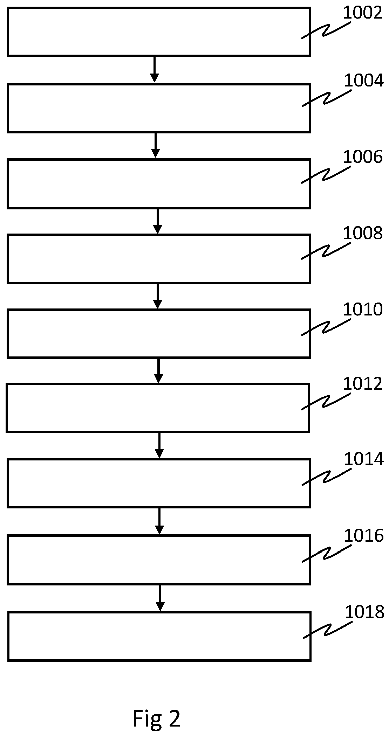

A flow diagram of a method of manufacturing a a sanitised paper envelope, is shown in Figure 2.

At step 1002, the first impression cylinder 6 is driven in an anti-clockwise direction (as viewed in Figure 1) such that the paper sheet 12 is continuously drawn from the roll of paper over the first guide roller, between the second guide roller 4b and onto the outer surface of the first impression cylinder 6.

As the first impression cylinder 6 rotates, the paper sheet 12 passes between the application roller 18a of the first sanitising module 10a and the first impression cylinder 6.

At step 1004, the sanitising solution is applied by the application roller 18a onto a first surface of the paper sheet 12, which is the surface facing radially outwardly from the first impression cylinder 6. The sanitising solution is applied to the paper sheet 12 by first drawing the sanitising solution from the fluid reservoir 20a onto the transfer roller 16a and then depositing the sanitising solution onto the application roller 18a which, in turn, deposits the sanitising solution onto the paper sheet 12. The first doctor blade 24a ensures that the thickness of the sanitising solution on the transfer roller 16a is a substantially constant predetermined thickness. This is done by 'scraping away' excess sanitising solution not in the cells, and so ensures that the amount of sanitising solution transferred to the application roller 18a, and subsequently applied to the paper sheet 12, is a predetermined amount. Once the sanitising solution has been applied, the paper sheet 12 continues to travel with the first impression cylinder 6 towards the first printing module 10b where it passes between the application roller 18b of the first printing module 10b and the first impression cylinder 6.

At step 1006, the first ink is applied by the application roller 18b onto the first surface of the paper sheet 12 by drawing the first ink from the fluid reservoir 20b via the transfer roller 16b and transferring the first ink to the application roller 18b which is then deposited by the application roller 18b onto the paper sheet 12. Once the first ink has been applied, the paper sheet 12 continues to travel with the first impression cylinder 6 towards the second printing module 10c where it passes between the application roller 18c of the second printing module 10c and the first impression cylinder 6.

At step 1008, the second ink is applied by the application roller 18c onto the first surface of the paper sheet 12 by drawing the second ink from the fluid reservoir 20c via the transfer roller 16c and transferring the second ink to the application roller 18c which is then deposited by the application roller 18c onto the paper sheet 12. Once the second ink has been applied, the paper sheet 12 continues to travel with the first impression cylinder 6 towards the third printing module 10d where it passes between the application roller 18d of the third printing module 10d and the first impression cylinder 6.

At step 1010, the third ink is applied by the application roller 18d onto the first surface of the paper sheet 12 by drawing the third ink from the fluid reservoir 20d via the transfer roller 16d and transferring the third ink to the application roller 18d which is then deposited by the application roller 18d onto the paper sheet 12. Once the third ink has been applied, the paper sheet 12 leaves the surface of the first impression cylinder 6 and travels around the third guide roller 4c and over the fourth and fifth guide rollers 4d, 4e and onto the outer surface of the second impression cylinder 8.

At step 1012, the sanitising solution is applied by the application roller 18e onto the second surface of the paper sheet 12 by drawing the sanitising solution from the fluid reservoir 20e via the transfer roller 16e and transferring the sanitising solution to the application roller 18e which is then deposited by the application roller 18e onto the paper sheet 12. Once the sanitising solution has been applied to the second surface, the paper sheet 12 leaves the outer surface of the second impression cylinder 8 and travels around the sixth and seventh guide rollers 4f, 4g before continuing to a cutting process.

The second doctor blade 24b, 24c, 24d, 24e of each of the respective printing modules 10b, 10c, 10d and sanitising module 10e ensures that the thickness of ink or sanitising solution on the respective transfer rollers 16a, 16b, 16c, 16d, 16e is a substantially constant predetermined thickness.

The process sanitises both sides of a paper sheet. By applying sanitising solution first the sanitising solution completely coats the first surface before it is then printed upon. Applying the sanitising solution first also means that the sanitising solution is transferred to other parts of the flexographic printing apparatus 2, such as the guide rollers 4a, 4b, 4c, 4d, 4e, 4f, 4g and the other application rollers 18a, 18b, 18c, 18d, 18e which helps to continuously sanitise the entire apparatus 2.

The second impression cylinder 8, application rollers 18a, 18b, 18c, 18d, 18e, transfer rollers 16a, 16b, 16c, 16d, 16e and guide rollers 4a, 4b, 4c, 4d, 4e, 4f, 4g are driven directly or indirectly by the first impression cylinder 6.

### Cutting and preparation

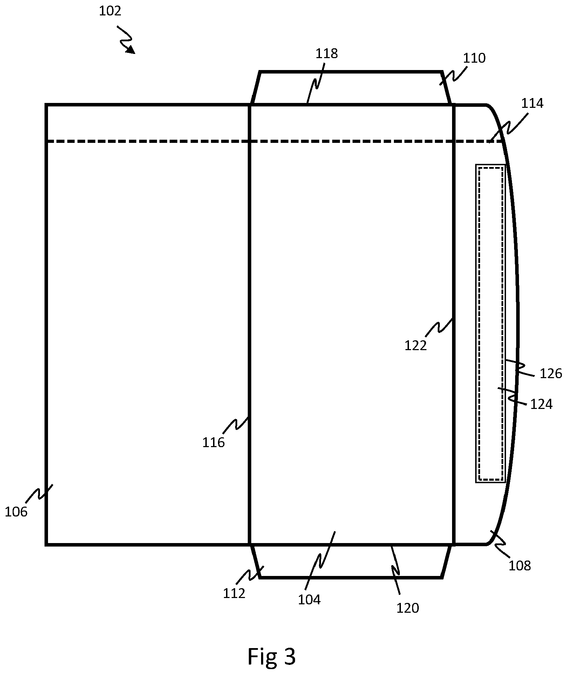

At step 1014, an envelope 'blank' 102 (see Figure 3) is cut from the paper sheet 12 by a cutter (not shown). The blank 102 has a rectangular front panel 104, a rectangular back panel 106 and a closure panel 108. An upper tab 110 is provided at the top of the front panel 104 and a lower tab 112 is provided at the bottom of the front panel 104. A perforation 114 extends across an upper portion of the blank 102 and parallel to the upper edges of the front panel 104, back panel 106 and closure panel 108.

The back panel 106 is joined to the front panel 104 at a first fold line 116. The upper tab 110 is joined to the front panel 104 at a second fold line 118 and the lower tab 112 is joined to the front panel 104 at a third fold line 120. The closure panel 108 is joined to the front panel 104 at a fourth fold line 122.

At step 1016, a sealing adhesive 124 is applied along the closure panel 108 and covered by a removable protective strip 126. Figure 3 shows the blank 102 arranged with the surface corresponding to the second surface of the paper sheet 12 from which it is cut facing upwardly.

### Assembly

At step 1018, an envelope 202 is assembled from the blank 102 by folding the upper and lower tabs 110, 112 inwardly along the respective second and third fold lines 118, 120 towards the front panel 104. An adhesive is applied to the upwardly facing surface of the upper and lower tabs 110, 112. The back panel 106 is then folded inwardly along the first fold line 116 towards the front panel 104 and brought into contact with the upper and lower tabs 110, 112. The adhesive is then allowed to cure, if needed.

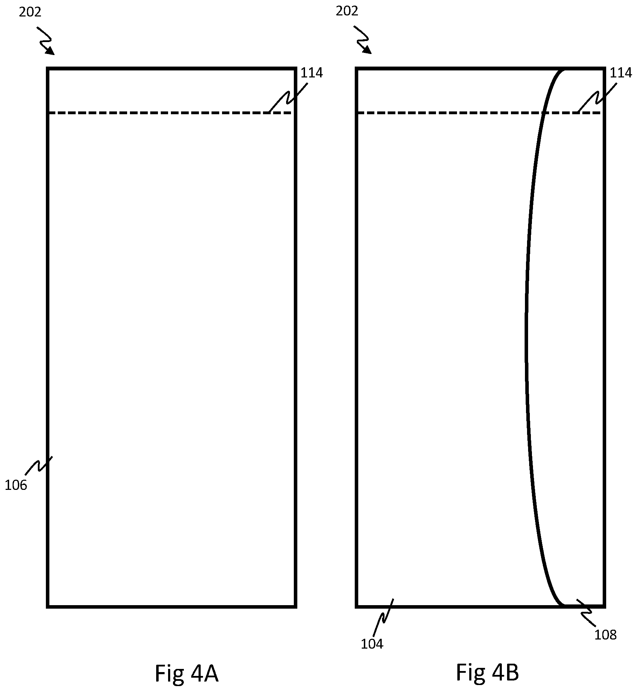

The finished envelope 202 has an inner surface, which corresponds to the second surface of the paper sheet 12, and an outer surface, which corresponds to the first surface of the paper sheet 12, which are sanitised. The assembled envelope 202 is shown from the front in Figure 4A and from the back in Figure 4B. The envelope 202 can then be packaged and transported for use.

The step of assembling the envelope 202 may be done mechanically by a machine. Assembly is carried out in a sanitised environment to avoid contamination of the envelope 202 prior to and during packaging for storage and transport. When assembled, the envelope 202 has a width of 110mm and a length of 240mm.

### Use

The envelope 202 is a cutlery envelope for storing cutlery temporarily after it has been cleaned and prior to being needed by a user. The envelope 202 is intended for use in catering, such as a restaurant or café, in which customers are provided with cutlery by the caterer.

After cutlery has been cleaned (e.g. washed and dried) it is clean and typically sterile. It is immediately inserted into the envelope 202, for example by a member of the catering staff, suitably attired through an aperture defined by the front and back panels 104, 106 in the vicinity of the fourth fold line 122. Any condiments, such as sachets of salt/pepper, and accessories, such as a napkin, can also be inserted into the envelope 202.

The protective strip 126 is then removed to expose the adhesive 124 underneath and the closure panel 108 is folded inwardly along the fourth fold line 122 so that the closure panel 108 is brought into contact with the back panel 106. The adhesive 124 secures the two together with an 'instant stick' destructive bond. The cutlery can then be stored in the envelope 202 until being given to a customer.

On receipt of the envelope 202 containing the cutlery, a customer tears along the perforation 114 to remove the upper end portion of the envelope 202. The cutlery, and any condiments and accessories, can then be removed by the customer.

A benefit of the envelope 202 is that it reduces the risk of contamination of cutlery with pathogens during transport and storage after washing of the cutlery and before use by a customer. In particular, having a sanitised envelope also ensures that pathogens are not transferred to the cutlery by the envelope 202 itself.

It will appreciated that the method described above could be used to manufacture other types of envelopes, such as envelopes for containing medical pills, or other paper articles such as packaging, notebooks or sketchpads.

## Claims

1. A method of manufacturing a sanitised paper envelope (202) comprising the steps:
supplying a paper sheet (12) having a first surface and a second surface to a flexographic printing apparatus (2) having a first sanitising module (10a);
applying a sanitising solution to at least the first surface of the paper sheet (12) using the first sanitising module (10a), and
forming an envelope (202) from the sanitised paper sheet (12).

2. The method of claim 1, wherein the flexographic printing apparatus (2) further comprises a second sanitising module (10e) and the method further comprises the step of applying a sanitising solution to the second surface of the paper sheet (12) using the second sanitising module (10e).

3. The method of claim 2, wherein the step of applying a sanitising solution to the second surface is subsequent to the step of applying a sanitising solution to the first surface.

4. The method of any one of the preceding claims, wherein the flexographic printing apparatus (2) further comprises at least one ink printing module (10b, 10c, 10d) and the method further comprises the step of printing a graphic and/or text on at least one of the first and second surfaces of the paper sheet (12).

5. The method of claim 4, wherein the step of printing on at least one of the first and second surfaces of the paper sheet (12) is subsequent to the step of applying a sanitising solution to at least the first surface of the paper sheet (12).

6. The method of any one of the preceding claims, wherein the sanitising solution comprises a sanitising agent and a carrier agent.

7. The method of claim 6, wherein the carrier agent is a liquid.

8. The method of claim 6 or 7, wherein the sanitising agent comprises ethanol.

9. The method of claim 8, wherein the sanitising solution comprises at least 70% ethanol.

10. The method of any one of the preceding claims, wherein the paper sheet (12) is supplied from a continuous roll of paper.

11. The method of claim 1, wherein the step of forming the envelope comprises the steps of:
cutting a blank (102) having a predetermined shape from the sanitised paper sheet (12), and
configuring said blank (102) to form a pocket having an aperture along one side.

12. The method of claim 11, wherein the blank (102) comprises a back panel (106), a front panel (104) and a closure panel (108) arranged such that the back panel (106) and the front panel (104) can be folded towards each other to form the pocket and the closure panel (108) can be folded over the aperture to inhibit access to the pocket.

13. The method of claim 12, further comprising a step of applying an adhesive covered by a removable protective strip to the blank (102) for securing the closure panel (108) over the aperture.

14. The method of any one of claims 1 to 13, further comprising a step of forming a perforation (114) extending across a region of the envelope (202) such that a user can remove a portion of the envelope (202) from the remaining envelope (202) by tearing along the perforation.

15. The method of any one of the preceding claims, wherein the or each sanitising module (10a) comprises a fluid supply unit (14a, 14e), an application roller (18a, 18e) for applying a sanitising solution to a paper sheet and a transfer roller (16a, 16e) arranged to transfer the sanitising fluid from the fluid supply unit (14a, 14e) to the application roller (18a, 18e).

16. The method of manufacturing of claim 15, wherein the sanitising module further comprises at least one doctor blade (22a, 22e) arranged to control the amount of sanitising solution transferred by the transfer roller (16a, 16e) to the application roller (18a, 18e).

17. A sanitised paper envelope (202) that has been manufactured in accordance with the method of any one of the preceding claims.

## Patentansprüche

1. Verfahren zur Herstellung eines sanitisierten Papierumschlags (202), das die folgenden Schritte umfasst:
Zuführen eines Papierbogens (12), der eine erste Oberfläche und eine zweite Oberfläche aufweist, zu einer Flexodruckeinrichtung (2), die ein erstes Sanitisierungsmodul (10a) aufweist;
Auftragen einer Sanitisierungslösung auf mindestens die erste Oberfläche des Papierbogens (12) unter Verwendung des ersten Sanitisierungsmoduls (10a) und
Bilden eines Umschlags (202) aus dem sanitisierten Papierbogen (12).

2. Verfahren nach Anspruch 1, wobei die Flexodruckeinrichtung (2) weiter ein zweites Sanitisierungsmodul (10e) umfasst und das Verfahren weiter den Schritt des Auftragens einer Sanitisierungslösung auf die zweite Oberfläche des Papierbogens (12) unter Verwendung des zweiten Sanitisierungsmoduls (10e) umfasst.

3. Verfahren nach Anspruch 2, wobei der Schritt des Auftragens einer Sanitisierungslösung auf die zweite Oberfläche auf den Schritt des Auftragens einer Sanitisierungslösung auf die erste Oberfläche folgt.

4. Verfahren nach einem der vorstehenden Ansprüche, wobei die Flexodruckeinrichtung (2) weiter mindestens ein Farbdruckmodul (10b, 10c, 10d) umfasst und das Verfahren weiter den Schritt des Druckens einer Grafik und/oder eines Textes auf mindestens eine der ersten und zweiten Oberfläche des Papierbogens (12) umfasst.

5. Verfahren nach Anspruch 4, wobei der Schritt des Bedruckens mindestens einer der ersten und zweiten Oberfläche des Papierbogens (12) auf den Schritt des Auftragens einer Sanitisierungslösung auf mindestens die erste Oberfläche des Papierbogens (12) folgt.

6. Verfahren nach einem der vorstehenden Ansprüche, wobei die Sanitisierungslösung ein Sanitisierungsmittel und einen Trägerstoff umfasst.

7. Verfahren nach Anspruch 6, wobei der Trägerstoff eine Flüssigkeit ist.

8. Verfahren nach Anspruch 6 oder 7, wobei das Sanitisierungsmittel Ethanol umfasst.

9. Verfahren nach Anspruch 8, wobei das Sanitisierungsmittel mindestens 70 % Ethanol umfasst.

10. Verfahren nach einem der vorstehenden Ansprüche, wobei der Papierbogen (12) von einer Endlospapierrolle zugeführt wird.

11. Verfahren nach Anspruch 1, wobei der Schritt des Bildens des Umschlags die folgenden Schritte umfasst:
Schneiden eines Zuschnitts (102), der eine vorbestimmte Form aufweist, aus dem sanitisierten Papierbogen (12) und
Konfigurieren des Zuschnitts (102) zum Bilden einer Tasche, die eine Öffnung entlang einer Seite aufweist.

12. Verfahren nach Anspruch 11, wobei der Zuschnitt (102) eine Rückwand (106), eine Vorderwand (104) und eine Verschlusswand (108) umfasst, die so angeordnet sind, dass die Rückwand (106) und die Vorderwand (104) aufeinander zu gefaltet werden können, um die Tasche zu bilden, und die Verschlusswand (108) über die Öffnung gefaltet werden kann, um den Zugang zur Tasche zu verhindern.

13. Verfahren nach Anspruch 12, weiter umfassend einen Schritt des Auftragens eines Klebstoffs, der von einem abnehmbaren Schutzstreifen bedeckt ist, auf den Zuschnitt (102) zur Befestigung der Verschlusswand (108) über der Öffnung.

14. Verfahren nach einem der Ansprüche 1 bis 13, weiter umfassend einen Schritt des Bildens einer Perforation (114), die sich über einen Bereich des Umschlags (202) erstreckt, so dass ein Benutzer einen Teil des Umschlags (202) von dem verbleibenden Umschlag (202) durch Aufreißen entlang der Perforation entfernen kann.

15. Verfahren nach einem der vorstehenden Ansprüche, wobei das oder jedes Sanitisierungsmodul (10a) eine Flüssigkeitszufuhreinheit (14a, 14e), eine Auftragswalze (18a, 18e) zum Auftragen einer Sanitisierungslösung auf einen Papierbogen und eine Übertragungswalze (16a, 16e) umfasst, die so angeordnet ist, dass sie die Sanitisierungsflüssigkeit von der Flüssigkeitszufuhreinheit (14a, 14e) zu der Auftragswalze (18a, 18e) überträgt.

16. Herstellungsverfahren nach Anspruch 15, wobei das Sanitisierungsmodul weiter mindestens eine Rakel (22a, 22e) umfasst, die so angeordnet ist, dass sie die Menge der Sanitisierungslösung steuert, die von der Übertragungswalze (16a, 16e) auf die Auftragswalze (18a, 18e) übertragen wird.

17. Sanitisierter Papierumschlag (202), der gemäß dem Verfahren nach einem der vorstehenden Ansprüche hergestellt wur

## Revendications

1. Procédé de fabrication d'une enveloppe en papier aseptisé (202) comprenant les étapes de :
alimentation en une feuille de papier (12) présentant une première surface et une seconde surface d'un appareil d'impression flexographique (2) présentant un premier module d'aseptisation (10a) ;
application d'une solution aseptisante sur au moins la première surface de la feuille de papier (12) en utilisant le premier module d'aseptisation (10a), et
formation d'une enveloppe (202) à partir de la feuille de papier aseptisé (12).

2. Procédé selon la revendication 1, dans lequel l'appareil d'impression flexographique (2) comprend en outre un second module d'aseptisation (10e) et le procédé comprend en outre l'étape d'application d'une solution aseptisante sur la seconde surface de la feuille de papier (12) en utilisant le second module d'aseptisation (10e).

3. Procédé selon la revendication 2, dans lequel l'étape d'application d'une solution aseptisante sur la seconde surface fait suite à l'étape d'application d'une solution aseptisante sur la première surface.

4. Procédé selon l'une quelconque des revendications précédentes, dans lequel l'appareil d'impression flexographique (2) comprend en outre au moins un module d'impression à encre (10b, 10c, 10d) et le procédé comprend en outre l'étape d'impression d'un graphique et/ou d'un texte sur au moins l'une des première et seconde surfaces de la feuille de papier (12).

5. Procédé selon la revendication 4, dans lequel l'étape d'impression sur au moins l'une des première et seconde surfaces de la feuille de papier (12) fait suite à l'étape d'application d'une solution aseptisante sur au moins la première surface de la feuille de papier (12).

6. Procédé selon l'une quelconque des revendications précédentes, dans lequel la solution aseptisante comprend un agent aseptisant et un agent vecteur.

7. Procédé selon la revendication 6, dans lequel l'agent vecteur est un liquide.

8. Procédé selon la revendication 6 ou 7, dans lequel l'agent aseptisant comprend de l'éthanol.

9. Procédé selon la revendication 8, dans lequel la solution aseptisante comprend au moins 70 % d'éthanol.

10. Procédé selon l'une quelconque des revendications précédentes, dans lequel la feuille de papier (12) est alimentée à partir d'un rouleau de papier continu.

11. Procédé selon la revendication 1, dans lequel l'étape de formation de l'enveloppe comprend les étapes de :
découpe d'une ébauche (102) présentant une forme prédéterminée à partir de la feuille de papier aseptisé (12), et
configuration de ladite ébauche (102) pour former une poche présentant une ouverture le long d'un côté.

12. Procédé selon la revendication 11, dans lequel l'ébauche (102) comprend un panneau arrière (106), un panneau avant (104) et un panneau de fermeture (108) agencés de sorte que le panneau arrière (106) et le panneau avant (104) puissent être pliés l'un vers l'autre pour former la poche et le panneau de fermeture (108) puisse être replié sur l'ouverture pour empêcher l'accès à la poche.

13. Procédé selon la revendication 12, comprenant en outre une étape d'application d'un adhésif recouvert d'une bande protectrice amovible sur l'ébauche (102) pour fixer le panneau de fermeture (108) sur l'ouverture.

14. Procédé selon l'une quelconque des revendications 1 à 13, comprenant en outre une étape de formation d'une perforation (114) s'étendant sur une région de l'enveloppe (202) de sorte qu'un utilisateur puisse enlever une partie de l'enveloppe (202) de l'enveloppe restante (202) en déchirant le long de la perforation.

15. Procédé selon l'une quelconque des revendications précédentes, dans lequel le ou chaque module d'aseptisation (10a) comprend une unité d'alimentation en fluide (14a, 14e), un rouleau applicateur (18a, 18e) pour appliquer une solution aseptisante sur une feuille de papier et un rouleau de transfert (16a, 16e) agencé pour transférer le fluide aseptisant de l'unité d'alimentation en fluide (14a, 14e) au rouleau applicateur (18a, 18e).

16. Procédé de fabrication selon la revendication 15, dans lequel le module d'aseptisation comprend en outre au moins une racle (22a, 22e) agencée pour réguler la quantité de solution aseptisante transférée par le rouleau de transfert (16a, 16e) au rouleau applicateur (18a, 18e).

17. Enveloppe en papier aseptisé (202) qui a été fabriquée conformément au procédé selon l'une quelconque des revendications précédentes.
